# EUROPÄISCHE PATENTANMELDUNG

(11) **EP 0 908 160 A2**
(43) Veröffentlichungstag der Anmeldung: **14.04.1999**
(21) Anmeldenummer: 98116721.6
(22) Anmeldetag: 03.09.1998
(51) Int. Cl.: A61F 2/36, A61F 2/46

(54) **Prothese**

(30) Priorität: 16.09.1997 DE 19740690
(71) Anmelder: Copf, Franz, D-70178 Stuttgart (DE)
(72) Erfinder: Copf, Franz, D-70178 Stuttgart (DE)
(74) Vertreter: Ostertag, Reinhard

(57) **Zusammenfassung**

Eine unter Zurhillfenahme von Zement einzugliedernde Prothese hat eine Tragplatte (24), die allseitig über einen Schaft (32) übersteht. Im Schaft ist ein axialer Speisekanal (36) für Zement ausgebildet, in einem überstehenden seitlichen Abschnitt (28) der Tragplatte (24) ist eine Saugöffnung (56) vorgesehen. Die distale Begrenzungsfläche der Tragplatte (24) trägt einen plastisch verformbaren Dichtrahmen (52). Auf diese Weise ist der Raum zwischen Corticalis-Innenfläche und Schaftaußenfläche dicht verschlossen und kann durch eine Saugpumpe (64) evakuiert werden. Hierdurch wird erreicht, daß sich der Zwischenraum zwischen Corticalis und Schaft einschlußfrei mit Zement füllt.

## Beschreibung

Der Erfindung betrifft eine Prothese gemäß dem Oberbegriff des Anspruches 1.

Bei einer derartigen Prothese gemäß der älteren Anmeldung 196 13 081.6 kann man auch bei axial sehr langen Kavitäten den Zwischenraum zwischen der Schaftaußenfläche und der Innenfläche der Corticalis des im Endabschnitt ausgeräumten Röhrenknochens gut mit dünnflüssigem Zement füllen, welcher den Zwischenraum zwischen Corticalis-Innenfläche und Schaftaußenfläche ausfüllt und so die Prothese fest mit dem Knochenende verbindet.

Trotzdem kommt es in manchen Anwendungen, insbesondere bei stark blutenden Kavitäten oder auch bei der Verwendung von Zement mit etwas höherer Viskosität zu Einschlüssen von Fremdflüssigkeit und/oder Luft. Hierdurch wird die Festigkeit der Verbindung zwischen Prothese und Knochenende beeinträchtigt.

Durch die vorliegende Erfindung soll daher eine Prothese gemäß dem Oberbegriff des Anspruches 1 so weitergebildet werden, daß auch unter den oben genannten ungünstigen Operationsbedingungen eine von Einschlüssen freie Zementschicht erhalten wird.

Diese Aufgabe ist erfindungsgemäß gelöst durch eine Prothese mit den im Anspruch 1 angegebenen Merkmalen.

Bei der erfindungsgemäßen Prothese ist durch das umlaufende Dichtelement der Tragplatte auch dann, wenn die Corticalis am Ende des Röhrenknochens nicht exakt gemäß der Kontur der Tragplatte reseziert wurde, was in der Praxis Schwierigkeiten bereitet, der Zwischenraum zwischen Corticalis-Innenfläche und Schaft-Außenfläche gegen die Umgebung abgedichtet. Man kann somit diesen Zwischenraum über die in der Tragplatte vorgesehene Saugöffnung ganz unter Unterdruck setzen, und man hat so in diesem Zwischenraum definierte Strömungsverhältnisse für den Zement, der von der Abgabeöffnung der Speisekanalanordnung abgegeben wird. Durch die Unterdruckbeaufschlagung werden Fremdflüssigkeiten und Luft auch zuverlässig abgesaugt, so daß man keine Einschlüsse in der Zementschicht erhält.

Die genannten Vorteile werden unter nur geringem Mehraufwand erhalten: in der Tragplatte muß nur eine zusätzliche Öffnung vorgesehen werden, und auf die distale Begrenzungsfläche der Tragplatte muß ein Dichtelement aufgesetzt werden.

Vorteilhafte Weiterbildungen der Erfindung sind in Unteransprüchen angegeben.

Mit der Weiterbildung der Erfindung gemäß Anspruch 2 wird die Abdichtung zwischen Tragplatte und Rand der Corticalis bleibend und unter geringem Kraftaufwand erhalten.

Besteht das Dichtelement aus hochvisköser Zementmasse, wie im Anspruch 3 angegeben, so erhält man nach dem Aushärten dieser Zementmasse eine zusätzliche Klebefläche zwischen dem freien Rand der Corticalis und der distalen Begrenzungsfläche der Tragplatte, was im Hinblick auf gute Kraftübertragung zwischen Prothese und Knochen von Vorteil ist.

Dabei vereinfacht die Weiterbildung der Erfindung gemäß Anspruch 3 die Handhabung der Prothese beim Operieren.

Auch mit der Weiterbildung der Erfindung gemäß Anspruch 5 erhält man eine gute Abdichtung zwischen der Tragplatte und dem Rand der Corticalis unabhängig von Ungenauigkeiten beim Resezieren der Corticalis.

Dabei ist die Weiterbildung der Erfindung gemäß Anspruch 6 dann vorteilhaft, wenn man das Dichtelement nach Ausfüllen des Zwischenraumes zwischen Corticalis-Innenfläche und Schaft-Außenfläche entfernen will. Es kann dann einfach zwischen den Rand der Corticalis und der distalen Begrenzungsfläche der Tragplatte herausgezogen werden. Der ursprünglich vom Dichtelement ausgefüllte Raum kann durch Nachfließen oder Nachpressen ebenfalls mit Zement ausgefüllt werden. Zusätzlich kann man auf die Prothese einen Schlag in distaler Richtung geben, damit die distale Begrenzungsfläche der Tragplatte so nahe wie möglich gegen den Rand der Corticalis bewegt wird.

Die Weiterbildung der Erfindung gemäß Anspruch 7 ist im Hinblick auf das Herausziehen des Dichtelementes aus dem Raum zwischen Corticalis und distaler Begrenzungsfläche der Tragplatte von Vorteil.

Die Weiterbildung der Erfindung gemäß Anspruch 8 ist im Hinblick auf ein vorläufiges axiales Festhalten der Prothese am Röhrenknochen von Vorteil, insbesondere dann, wenn man mit einem elastischen Dichtelement arbeitet, welches den Spalt zwischen distaler Begrenzungsfläche der Tragplatte und Rand der Corticalis unter unterschiedlicher lokaler elastischer Verformung in Dickenrichtung ausgleicht.

Mit der Weiterbildung der Erfindung gemäß Anspruch 9 wird erreicht, daß die Saugöffnung bis zum letzten Teil des Füllen des Zwischenraumes zwischen Schaft-Außenfläche und Corticalis-Innenfläche funktionsfähig ist.

Die Weiterbildungen der Erfindung gemäß den Ansprüchen 10 und 11 sind im Hinblick auf ein leichtes Herstellen und Lösen einer dichten Verbindung zwischen Anschlußleitungen und Tragplatte von Vorteil.

Nachstehend wird die Erfindung anhand von Ausführungsbeispielen unter Bezugnahme auf die Zeichung näher erläutert. In dieser zeigen:
- Figur 1:: einen Längsschnitt duch das Ende eines Oberschenkelknochens mit einer in dieses eingeführten Oberschenkelprothese, gezeigt unter Operationsbedingungen;
- Figur 2:: eine axiale Aufsicht auf das distale Ende eines Schaftes der in Figur 1 gezeigten Prothese; und
- Figur 3:: eine ähnliche Ansicht wie Figur 3, in welcher jedoch eine abgewandelte Prothese wiedergegeben ist.

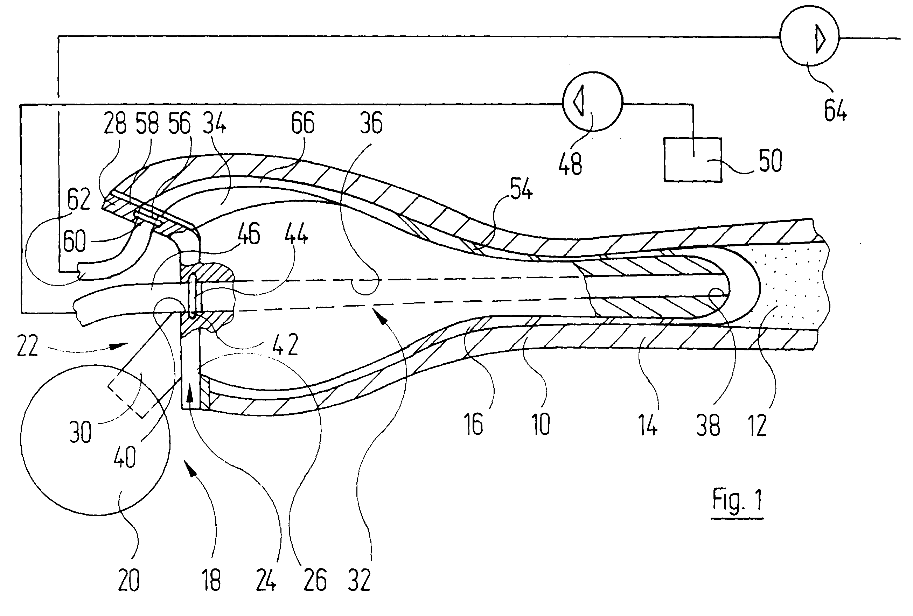

In Figur 1 ist bei 10 der obere Endabschnitt eines Femurs wiedergegeben. Der die Gelenkkugel tragende Trochander minor ist reseziert, wobei die Resektionsfläche im wesentlichen senkrecht auf der Achse des Oberschenkelknochens steht und in einem in Figur 1 oben liegenden Abschnitt nach proximal hin ansteigt.

Proximal entspricht in Figur 1 nach links, distal in Figur 1 nach rechts. In der nachstehenden Beschreibung werden ferner die Richtungsbezeichnungen lateral und medial sowie dorsal und ventral verwendet. Lateral bedeutet in Figur 1 nach oben, medial in Figur 1 nach unten. Dorsal bedeutet in Figur 1 senkrecht zur Zeichenebene nach hinten, ventral in Figur 1 senkrecht zur Zeichenebene nach vorne.

Vom resezierten Ende des Femurs 10 ist die Spongiosa, die im rechten Teil des Oberschenkelknochens noch bei 12 gezeigt ist, ausgeräumt, so daß die außenliegende Corticalis 14 eine Kavität 16 begrenzt.

In die Kavität 16 ist eine insgesamt mit 18 bezeichnete Prothese eingesetzt. Diese umfaßt eine Gelenkkugel 20, die aus Keramik hergestellt ist und an der Oberfläche poliert ist, sowie einen insgesamt mit 22 bezeichneten Prothesenkörper.

Letzterer umfaßt eine abgewinkelte Tragplatte 24 mit einem senkrecht auf der Femurachse stehenden Hauptabschnitt 26 und einem in Proximalrichtung gekippten Seitenabschnitt 28. Gemäß der Kontur der Tragplatte 24 wurde die Resektion des Knochenendes vorgenommen. Die Randkontur der Tragplatte 24 entspricht der Randkontur der Corticalis 14 bei der Resektionsfläche.

Die proximale Begrenzungsfläche der Tragplatte 24 trägt einen angeformten Tragzapfen 30 auf welchem die Gelenkkugel 20 befestigt ist.

Die distale Begrenzungsfläche der Tragplatte 24 trägt einen Schaft 32, dessen Mantelfläche der Innenfläche der Corticalis unter kleinem Abstand folgt. Wie aus der Zeichnung ersichtlich, vermindert sich der transversale Querschnitt des Schaftes 32 zu seinem distalen Ende hin.

Der transversale Querschnitt des Schaftes 32 kann in der Praxis in etwa einem Rechteck mit abgerundeten Ecken entsprechen. Im Bereich des stehengebliebenen Teiles des Trochander major ist auf die Außenfläche des Schaftes 32 eine den Trochanderverlauf nachahmende Rippe 34 aufgesetzt.

Durch die Tragplatte 24 und den Schaft 32 erstreckt sich eine Speisekanal 36, der die Endfläche des Schaftes 32 schneidet und dort eine Abgabeöffnung 38 vorgibt. Der Speisekanal 36 tritt proximal in die Tragplatte 24 aus und gibt dort eine Speiseöffnung 40 vor. In der Speiseöffnung 40 ist eine Ringnut 42 eingestochen, welche eine Ringwulst 44 unter elastischer Kompression aufnehmen kann, welche im Endabschnitt eines Speiseschlauches 46 vorgesehen ist. Letzterer führt zur Förderseite einer Zement-Speisepumpe 48, die aus einem Zementvorratsbehälter 50 ansaugt.

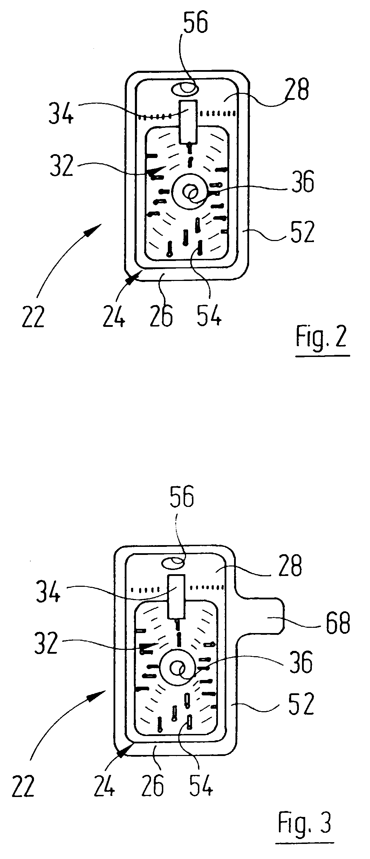

Wie insbesondere aus Figur 2 ersichtlich, steht die Tragplatte 24 allseitig über den Schaft 32 über. Ihr Rand ist bündig zur Außenfläche der Corticalis 14, wie schon dargelegt. In dem der durch Resektion erhaltenen freien Stirnfläche der Corticalis 14 gegenüberliegenden Randbereich trägt die Tragplatte 24 einen umlaufenden Dichtrahmen 52. Dieser besteht beim in den Figuren 1 und 2 gezeigten Ausführungsbeispiel aus einer Schicht aus hochviskosem Zementmaterial. Es kann sich dabei um das gleiche Zementmaterial handeln, welches zum Verbinden des Schaftes 32 mit der Innenfläche der Corticalis 14 verwendet wird, wobei dem Dichtrahmenmaterial zusätzliche Füllstoffe zugesetzt sind und/oder die Zusammensetzung des Zementes derart abgewandelt ist, daß die Aushärtung langsamer erfolgt. In diesem Falle kann dann das hochviskose Zementmaterial auch schon teilweise ausgehärtet oder vorgetrocknet sein. In der Praxis kann die Dicke des Dichtrahmens 52 etwa 1 bis 5 mm, vorzugsweise 2 bis 4 mm betragen. Damit kann der Dichtrahmen 52 die bei der Resektion nicht vermeidbaren Konturabweichungen zwischen der freigelegten Stirnfläche der Corticalis und der distalen Begrenzungsfläche der Tragplatte 24 ausgleichen. Beim Hineinschieben der Prothese 18 in die Kavität 16 wird überschüssiges Material des Dichtrahmens 52 verdrängt.

Um die durch Einschieben der Prothese in die Kavität erhaltene Lage zu sichern, trägt die Mantelfläche des Schaftes 32 nach distal geneigte kurze Drahtstücke 54, deren Länge in der Praxis 5 mm betragen kann. Der Anstellwinkel der Drahtstücke 54 zur Schaftachse kann in der Praxis etwa 60° betragen. Durch elastisches Biegen der Drahtstücke in Richtung distal können sich die Enden der Drahtstücke 54 der Ist-Kontur der Corticalis-Innenfläche anpassen. Die Stirnflächen der Drahtstücke verhaken sich mit Rauhigkeiten an der Corticalis-Innenfläche bzw. arbeiten sich in von der Corticalis-Innenfläche getragene Restschichten an Spongiosa hinein. Die Drahtstücke 54 bilden somit in distaler Richtung wirksame Widerhaken.

Im Seitenabschnitt 28 der Tragplatte ist eine Saugöffnung 56 vorgesehen. In der Saugöffnung 56 ist eine umlaufende Ringnut 58 vorgesehen, die mit einem komplementären elastisch verformbaren Ringwulst 60 am Ende eines Saugschlauches 62 zusammenarbeitet. Letzterer führt zur Saugseite einer Saugpumpe 64.

Die oben beschriebene Prothese wird wie folgt implantiert:

Nach Schaffung der Kavität 16 wird die Prothese 18 in distaler Richtung in die Kavität 16 hineingefahren, wobei auf dem letzten Teil des Weges der von der distalen Begrenzungsfläche der Tragplatte 24 getragene Dichtrahmen 52 entsprechend der Ist-Kontur des Corticalisrandes plastisch verformt wird. Die Tragplatte 24 sitzt am Ende des Einführvorganges mit einigen Teilbereichen direkt auf den vor der idealen Resektionsfläche liegenden Bereichen des Randes des Corticalis auf. Die zwischen dem Rest der distalen Begrenzungsfläche der Tragplatte 24 und der Stirnfläche der Corticalis 14 verbleibenden Zwischenräume schließt der Dichtrahmen 52. Beim Einführen in die Kavität 16 wurden die Drahtstücke 54 zusätzlich gebogen und haben sich an der Corticalis-Innenfläche verhakt. Auf diese Weise ist die Position der Prothese 18 im Knochenende vorläufig fixiert. Nun werden der Speiseschlauch 46 und Saugschlauch 52 an die Speiseöffnung 40 bzw. die Saugöffnung 56 angeschlossen. Der zwischen der Schaft-Außenfläche und der Corticalis-Innenfläche liegende Raum 66 wird nun durch die Saugpumpe 64 evakuiert. Hierbei wird Flüssigkeit, die sich in der Kavität 16 angesammelt hat, abgesaugt. Nun wird die Zementzufuhr über den Speiseschlauch 46 eingeschaltet und die Kavität 16 wird vom distalen Ende her mit dünnflüssigem Zement versorgt. Der aus der Abgabeöffnung 38 fließende Zement läuft in der Kavität 16 unter Schwerkraft zunächst nach unten und füllt die Kavität von unten nach oben an. Dieses Füllen der Kavität erfolgt unter weiterem ständigen Absaugen, so daß vor der Zementoberfläche liegende Flüssigkeitsmengen zuverlässig abgesaugt werden und Lufteinschlüsse verhindert werden. Zum Ende des Füllvorganges tritt dann flüssiger Zement in den Saugschlauch 62 ein. Zu diesem Zeitpunkt kann dann das Füllen beendet werden.

Nach Aushärten des Zementes kann man dann den Speiseschlauch 46 und den Saugschlauch 62 direkt an der proximalen Begrenzungsfläche der Tragplatte abtrennen.

Bei dem abgewandelten Ausführungsbeispiel nach Figur 3 ist der Dichtrahmen 52 aus einem elastomeren Material hergestellt. Er ist durch eine leicht aufbrechbare biokompatible Klebstoffschicht oder durch Aufspiessen auf dünne und kurze distale Nadeln der Tragplatte an letztere angeheftet und somit mit der Prothese einstückig handhabbar.

Am Rand des Dichtrahmens 52 ist eine Greifflasche 68 vorgesehen, an der der Dichtrahmen 52 nacn Füllen des Raumes 66 radial nach außen und über den Rand der Tragplatte 24 in Proximalrichtung gezogen werden kann. Nach Durchschneiden kann der Dichtrahmen 52 dann entfernt werden.

In Abwandlung der obigen Ausführungsbeispiele kann man als Material für den Dichtrahmen auch plastische verformbare Keramik-Schaummaterialien, plastisch verformbare Metalle oder Metallschäume oder plastisch verformbare Kunststoff-Schaummaterialien oder entsprechende plastisch verformbare Sintermaterialien aus den genannte Werkstoffen einsetzen.

## Patentansprüche

1. Prothese mit einem gestreckten Schaft (32), der in eine in einem Endabschnitt des Röhrenknochens (10) ausgeräumte Kavität einführbar ist und eine Speisekanalanordnung (36) aufweist, die mindestens eine in die Mantelfläche und/oder die Stirnfläche des Schaftes (32) ausmündende Abgabeöffnung (38) und eine in die Tragplatte (24) ausmündende Speiseöffnung (40) aufweist, wobei die Tragplatte (24) zumindest in einem Teilbereich ihres Randabschnittes über den Schaft (32) übersteht, dadurch gekennzeichnet, daß in dem überstehenden Bereich des Randabschnittes eine durch die Tragplatte (24) hindurch gehende Saugöffnung (56) vorgesehen ist und die distale Begrenzungsfläche der Tragplatte (24) ein umlaufendes Dichtelement (52) trägt.

2. Prothese nach Anspruch 1, dadurch gekennzeichnet, daß das Dichtelement (52) plastisch verformbar ist.

3. Prothese nach Anspruch 2, dadurch gekennzeichnet, daß das Dichtelement (52) eine hochvisköse Zementmasse aufweist.

4. Prothese nach Anspruch 3, dadurch gekennzeichnet, daß die Zementmasse vorgetrocknet oder vorgehärtet ist.

5. Prothese nach Anspruch 2, dadurch gekennzeichnet, daß das Dichtelement (52) ein Schaummaterial oder ein Sintermaterial aus vorzugsweise gewebekompatiblem anorganischem Material, z.B. Keramikschaummaterial, oder aus gewebekompatiblem organischem Material, z.B. Polyäthylenschaummaterial besteht.

6. Prothese nach Anspruch 1 oder 2, dadurch gekennzeichnet, daß das Dichtelement (52) aus zähem auf Zug belastbarem Material hergestellt ist, z.B. einem elastomerem Material.

7. Prothese nach Anspruch 6, dadurch gekennzeichnet, daß das Dichtelement (52) eine radial nach außen überstehende Greiflasche (68) aufweist.

8. Prothese nach einem der Ansprüche 1 bis 7, dadurch gekennzeichnet, daß die Außenfläche des Schaftes (32) eine Mehrzahl nach proximal schräg angestellter Halteelemente (54) aufweist, vorzugsweise in Form von Drahtstücken.

9. Prothese nach einem der Ansprüche 1 bis 8, dadurch gekennzeichnet, daß die Saugöffnung (56) in demjenigen Bereich der Tragplatte (24) vorgesehen ist, welcher unter Operationsbedingungen am weitesten oben liegt.

10. Prothese nach einem der Ansprüche 1 bis 9, dadurch gekennzeichnet, daß eine Speiseleitung (46) für Zement und/oder eine Saugleitung (62) Rastmittel (44, 60) aufweisen, die mit der Tragplatte (24) zusammenarbeiten.

11. Prothese nach Anspruch 10, dadurch gekennzeichnet, daß die Rastmittel die Form eines elastischen Ringwulstes (44, 60) haben, die mit einer Ringnut (42, 58) der Speiseöffnung (40) bzw. der Saugöffnung (56) zusammenarbeitet.
